# EUROPEAN PATENT SPECIFICATION

(11) **EP 1 547 557 B1**
(45) Date of publication and mention of the grant of the patent: **05.10.2011**
(21) Application number: 03791213.6
(22) Date of filing: 13.08.2003
(51) Int. Cl.: A61F 13/496

(54) **UNDERPANTS TYPE DISPOSABLE DIAPER**
EINWEG-WINDEL VOM HÖSCHENTYP
COUCHE JETABLE DE TYPE CULOTTE

(30) Priority: 30.08.2002 JP 2002252969; 14.07.2003 JP 2003196206
(43) Date of publication of application: 29.06.2005
(73) Proprietor: UNI-CHARM CO., LTD., Kawanoe, Ehime 799-0111 (JP)
(72) Inventor: OTSUBO, Toshifumi, Tech. Ctr., Uni-Charm Co., Ltd., Mitoyo-gun, Kagawa 769-1602 (JP); TAKINO, Shunsuke, Technical Ctr., Uni-Charm Co Ltd, Mitoyo-gun, Kagawa 769-1602 (JP)
(74) Representative: Sperling, Rüdiger
(86) International application number: PCT/JP2003/010312
(87) International publication number: WO 2004/019843

(56) References cited:
- EP-A- 1 486 189
- EP-A- 1 495 739
- JP-A- 11 188 062
- JP-A- 50 021 845
- JP-A- 50 033 044
- JP-A- 2002 035 033
- JP-A- 2003 010 244
- JP-A- 2003 220 091
- JP-A- 2003 230 594

## Description

### TECHNICAL FIELD OF THE INVENTION

This invention relates to a pull-on disposable diaper.

### RELATED ART

Japanese Patent Application No. 1975-33044A discloses a foldup-type disposable diaper 201 as shown in Fig. 10 of the accompanying drawings. This diaper 201 is composed of a liquid-absorbent pad, a liquid-pervious inner sheet 222 and a liquid-impervious outer sheet 223 so as to have a rectangle shape and then folded along a transversal 210a orthogonal to long sides of the diaper 201 in two halves in a longitudinal direction. Simultaneously, the diaper 201 is tucked from its transversely opposite edges inwardly of the diaper, more specifically, toward a middle point of the transversal 210a so as to form pockets 218. This diaper 201 is adapted to be worn in combination with use of a diaper cover and surface sections of the sheet 223 facing each other as the diaper 201 is tucked inward in this manner are partially joined in order to prevent the respective pockets 218 might get out of initial shapes thereof even after the diaper 201 has been developed to be put on a wearer' s body. The diaper arranged in such manner is effective to avoid leakage of body fluids regardless of its rectangular shape because a region of the diaper destined to cover the wearer's crotch is sufficiently narrow to be placed closely against the wearer's crotch.

Japanese Utility Model Application No. 1972-36734A discloses a foldup-type diaper made from a rectangular strip. This diaper also is adapted to be worn in combination with use of a diaper cover and the diaper is tucked inward from its transversely opposite edges in a longitudinally middle zone of the diaper. The crotch region of the diaper obtained in this manner has its width sufficiently reduced to be placed closely against the wearer's crotch and thereby to alleviate an anxiety of sideways urine leakage.

The diaper disclosed in Japanese Patent Application No. 1975-33044A intends to prevent the diaper from getting out of its initial shape by partially joining together the surface sections opposed to each other as the diaper is folded and tucked. However, such joining may obstruct the diaper to be flatly developed and retard operation of putting the diaper on the wearer's body.

The diaper disclosed in the above-cited Japanese Utility Model Application No. 1972-36734A is accompanied with an inconvenience that the crotch region folded and tucked in this manner may get out of its desired shape as the diaper is put on the wearer's body.

It is an object of this invention to provide a pull-on disposable diaper having a body fluid absorbing component partially folded inward in a crotch covering region improved so that zones of the body fluid absorbing component folded inward can be reliably maintained in such a folded state even if these folded zones are not joined such as in the prior art.

### DISCLOSURE OF THE INVENTION

In accordance with this invention, there is a pull-on disposable diaper having a height direction, a width direction being orthogonal to the height direction and a back-and-forth direction, the diaper being substantially symmetric about a center line bisecting a dimension of the diaper in the width direction, the diaper comprising a covering component made of sheet material having an inner surface facing a wearer's body and outer surface facing a wearer's clothes and composed of front waist covering region destined to cover a wearer's front waist, a rear waist covering region destined to cover a wearer' s rear waist and a crotch covering region destined to cover a wearer's crotch so as to define a pants-like configuration having a waist-hole and a pair of leg-holes, and a body fluid absorbing component extending over the crotch covering region and further extending into the front and rear waist covering regions.

The pull-on disposable diaper further comprises the body fluid absorbing component comprising a liquid-absorbent core and a liquid-pervious cover sheet. The core have an inner surface facing the wearer's body, an outer surface facing away from the wearer's body and side surfaces extending between the inner and outer surfaces in the height direction and extending beyond the crotch covering region into the front and rear waist covering regions. The cover sheet covers the inner surface, the side surfaces and at least a part of the outer surface of the core. The body fluid absorbing component is formed in the crotch covering region with a first folding guide extending from a middle zone between transversely opposite lateral edges extending parallel to each other in the height direction to the respective lateral edges in the front waist covering region so as to describe a substantially V-shape, a second folding guide extending to the respective lateral edges in the rear waist covering region so as to describe a substantially V-shape and a third folding guide, extending in the width direction between the first and second folding guides. The absorbing component is folded on both sides of the center line along the third folding guide so that the core has its outer surface sections facing to each other and along the first and second folding guides so that the core has its inner surface sections facing to each other, transversely opposite lateral edges of the cover sheet is folded back outward in the width direction on the outer surface of the core and is joined to the inner surface of the sheet material defining the crotch covering region.

This invention includes the following embodiments.

The outer surface of the core is covered at least with the cover sheet and a liquid-impervious sheet lying outside the cover sheet and defining the crotch covering region.

The outer surface of the core is covered with the cover sheet, a nonwoven fabric lying outside the cover sheet, a plastic film lying outside the nonwoven fabric and defining the liquid-impervious sheet and a nonwoven fabric lying outside the liquid-impervious sheet.

### BRIEF DESCRIPTION OF THE DRAWINGS

Fig. 1 is a perspective view showing a pants-type disposable diaper;
Fig. 2 is a partially cutaway perspective view showing the diaper of Fig. 1;
Fig. 3 is a sectional view taken along a line III-III in Fig. 1;
Fig. 4 is a developed view of the diaper of Fig. 1;
Fig. 5 is a sectional view taken along a line V-V in Fig. 4; and

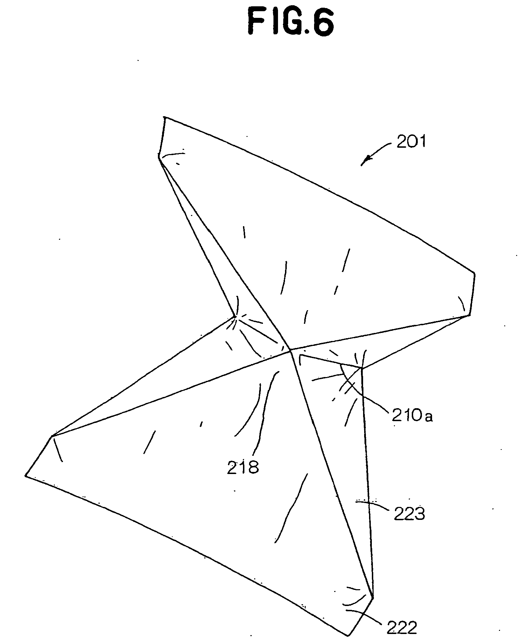

Fig. 6 is a perspective view showing an example of the conventional diaper.

### DESCRIPTION OF THE BEST MODE FOR WORKING OF THE INVENTION

Details of the pull-on disposable diaper according to this invention will be more fully understood from the description given hereunder with reference to the accompanying drawings.

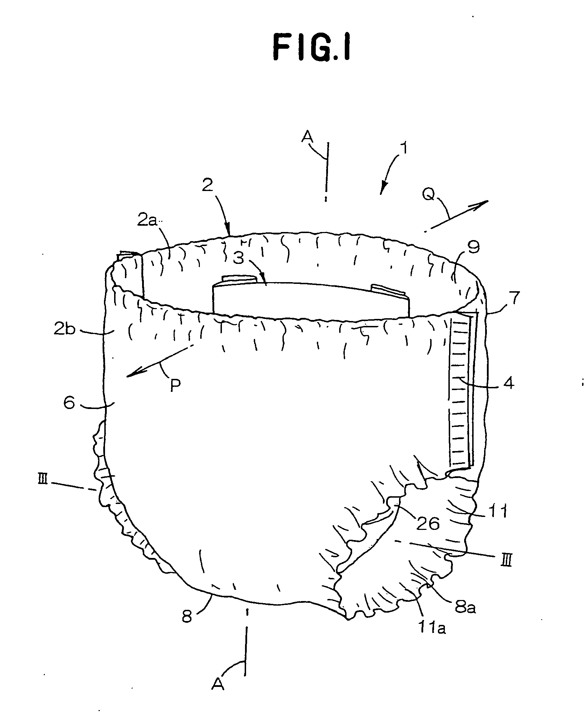

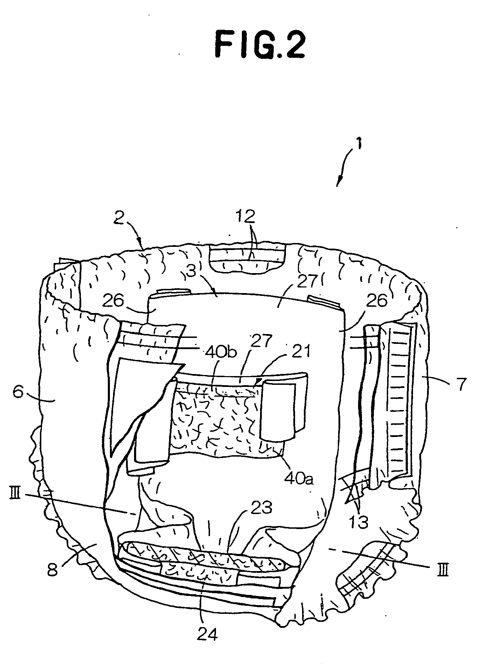

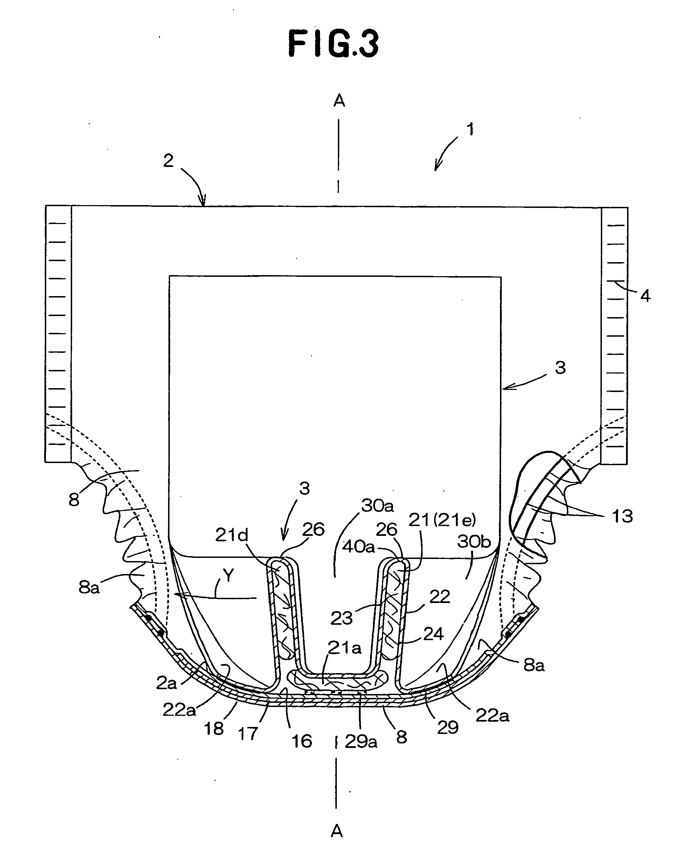

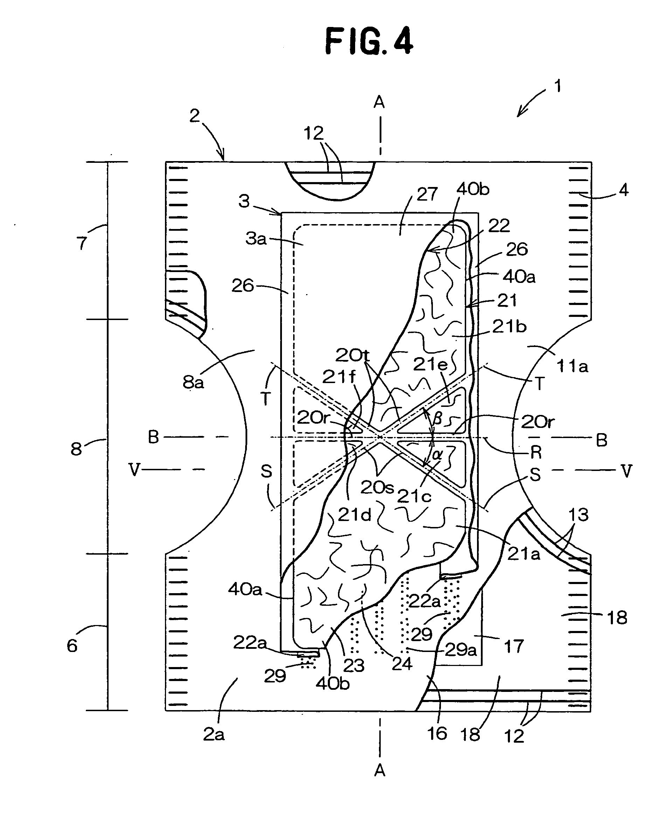

Fig. 1 is a perspective view showing a pull-on disposable diaper 1, Fig. 2 is a partially cutaway perspective view showing this diaper 1 and Fig. 3 is a sectional view taken along a line III-III in Fig. 1. The diaper 1 has height and width directions orthogonal to each other and a back-and-forth direction. The height direction corresponds to a vertical direction in Fig. 1, the back-and-forth direction corresponds to a direction indicated by a double-headed arrow P-Q in Fig. 1 and the width direction corresponds to a right-and-left direction as viewed in Fig. 3. The diaper 1 basically comprises a pants-like covering component 2 and a body fluid absorbing component 3 for containment of body fluids absorbed therein. The covering component 2 is formed by sheet material such as a nonwoven fabric or plastic film and has an inner surface 2a facing a diaper wearer' s body and an outer surface 2b facing the wearer' s clothes. This covering component 2 is composed of a front waist covering region 6, a rear waist covering region 7 and a crotch covering region 8 adapted to cover wearer's front, rear and crotch regions, respectively. The front and rear waist covering regions 6, 7 are overlaid together along transversely opposite lateral marginal edges of the diaper 1 and joined together along these opposite lateral marginal edges at a plurality of joining zones 4 arranged intermittently in a vertical direction along these opposite lateral marginal edges so that the front waist covering region 6, the rear waist covering region 7 and the crotch covering region 8 cooperate with one another to define a waist-hole 9 and a pair of leg-holes 11. The waist-hole 9 and the leg-holes 11 are provided along peripheral marginal edges thereof with a waist-circumferential elastic member 12 and leg-circumferential elastic members 13 attached in a stretched state thereto, respectively. The body fluid absorbing component 3 lies on the inner surface 2a of the covering component 2 so as to extend over the crotch covering region 8 and to extend further into the front and rear waist covering regions 6, 7. The body fluid absorbing component 3 is folded toward a longitudinal center line A - A (See Fig. 4 also) bisecting a width of the diaper 1, i.e., folded inward in a transverse direction of the diaper 1 so that the body fluid absorbing component 3 may have its width reduced in the crotch covering region 8.

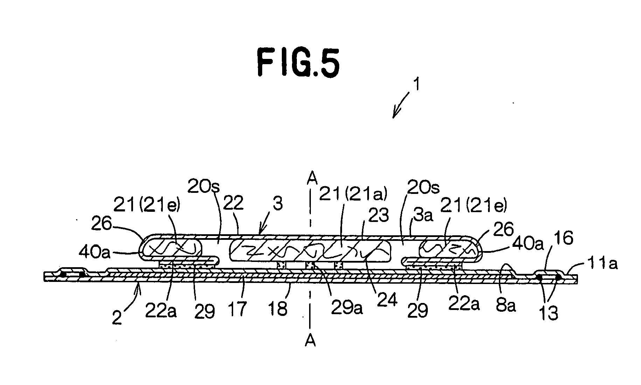

Fig. 4 is a partially cutaway plan view showing the diaper 1 of Fig. 1 in which front and rear waist regions 6, 7 have been disconnected from each other at the joining zones 4 and developed in directions indicated by the arrows P, Q extending in the back-and-forth direction and Fig. 5 is a sectional view taken along a line V-V in this plan view. As shown, the developed diaper 1 has its length as viewed in the vertical direction of Fig. 3 bisected by a transverse center line B - B extending in a direction which is orthogonal to the longitudinal center line A - A. In the developed state as shown in Fig. 4, the diaper 1 is substantially symmetric about the longitudinal center line A - A and may be folded back along the transverse center line B - B to obtain the diaper 1 in the state as shown in Fig. 1.

The covering component 2 comprises an hourglass-shaped inner sheet 16, a rectangular intermediate sheet 17 lying on the outer side of the inner sheet 16 and an outer sheet 18 lying on the outer side of the intermediate sheet 17. The inner sheet 16 is preferably made of breathable nonwoven fabric, more preferably made of hydrophobic breathable nonwoven fabric. The intermediate sheet 17 is preferably made of liquid-impervious plastic film, more preferably made of breathable but liquid-impervious plastic film and identical in shape as well as in size to the absorbing component 3. The outer sheet 18 is made of breathable nonwoven fabric and identical in shape as well as in size to the inner sheet 16. These sheets 16, 17, 18 are intermittently joined to one another by adhesion or heat-sealing to form the covering component 2. The nonwoven fabric layers forming the inner and outer sheets 16, 18 of the covering component 2 contribute to a soft touch of the diaper 1. The waist- and leg-circumferential elastic members 12, 13 interposed between the inner and outer sheets 16, 18 are attached to at least one of these sheets 16, 18 by means of an adhesive agent (not shown).

The absorbing component 3 comprises a core 21 and a cover sheet 22 and has a rectangular shape which is relatively long in the vertical direction as viewed in Fig. 4. This rectangular absorbing component 3 is contoured by transversely opposite lateral edges 26 extending parallel to the longitudinal center line A - A and front and rear ends 27 extending in the transverse direction of the diaper 1 orthogonal to the transversely opposite lateral edges 26. The core 21 has a rectangular shape as a whole and has an inner surface 23 facing the wearer's body, an outer surface 24 facing the wearer's clothes, opposite side surfaces 40a extending in the longitudinal direction and opposite end surfaces 40b extending across the front and rear waist covering regions 6, 7 in the transverse direction. The core 21 additionally has grooves 20r, 20s, 20t (See Fig. 5 also) extending along a chain line R (third folding guide) extending in coincidence with the transverse center line B - B, a chain line S (first folding guide) extending so as to describe V-shape from the central zone of the core 21 defined by the intersection of the longitudinal center line A - A and the transverse center line B - B toward the front waist covering region 6 to the opposite side surfaces 40a and a chain line T (second folding guide) extending so as to describe V-shape from the central zone of the core 21 defined by the intersection of the longitudinal center line A - A and the transverse center line B - B toward the rear waist covering region 7 to the opposite side surface 40a. These grooves 20r, 20s, 20t divide the core 21 into core elements 21a, 21b, 21c, 21d, 21e, 21f. The groove 20r and the groove 20s intersect with each other at an angle α while the groove 20r and the groove 20t intersect with each other at an angle β. While the angles α and β are illustrated to be equal to each other, it is possible without departing from the scope of the invention to set these two angles to be different from each other. In the crotch covering region 8, the absorbing component 3 is narrower than this crotch covering region 8, so lateral marginal edges 8a of the crotch covering region 8 form leg-circumferential flaps 11a extending outward beyond the lateral edges 26 of the crotch covering region 8, which define the respective leg-holes 11.

The core 21 divided in the core elements 21a through 21f is formed by compressing water-absorbent material such as a fluff pulp and super-absorbent polymer particles under an appropriate pressure and, if desired, covering such a compressed material with a tissue paper or a nonwoven fabric of thermoplastic synthetic fibers modified to be hydrophilic. The inner surface 23 of the core 21 divided in the core elements 21a through 21f or the tissue paper or the like (not shown) covering this inner surface 23 may be joined to the cover sheet 22. The outer surface 24 of the core 21 or the tissue paper or the like covering this outer surface 24 may be joined to the inner sheet 16 by means of an adhesive agent 29a (See Fig. 5). The cover sheet 22 extends over the inner surface 23 in the transverse direction of the core 21 and reaches to the outer surface 24 in the vicinity of the respective side surfaces 40a beyond the side surfaces 40a. On the outer surface 24, lateral edge zones 22a of the cover sheet 22 are folded outward in the transverse direction of the core 21 and these lateral edge zones 22a folded in this manner are joined to the inner surface of the inner sheet 16 by means of a hot melt adhesive agent 29. Portions of the cover sheet 22 extending beyond the end surfaces 40b of the core 21 are bonded to the inner sheet 16 and layers of the sheet 22 overlaying each other are bonded together in the vicinity of these portions using a hot melt adhesive agent (not shown). Such cover sheet 22 is folded in a Z-shape or in an inverted Z-shape in the vicinity of the side surfaces 40a of the core 21 (See Figs. 4 and 5). A stock material for the cover sheet 22 may be selected from the group consisting of a liquid-pervious nonwoven fabric, a perforated plastic film and a laminated sheet of the nonwoven fabric and the film.

The diaper 1 having the absorbing component 3 formed in this manner is folded back from the developed state shown in Fig. 4 along the transverse center line B - B and then the front and rear waist covering regions 6, 7 are connected to each other at the joining zones 4 to obtain the diaper 1 of Fig. 1. In this course, respective halves of the absorbing component 3 lying on both sides of the longitudinal center line A - A are folded toward this longitudinal center line A - A as shown in Figs. 2 and 3. Specifically, the absorbing component 3 is folded along the groove 20r so that the outer surface 24 of the core element 21c is opposed to the outer surface 24 of the 21e (See Fig. 5) while the outer surface 24 of the core element 21d is opposed to the outer surface 24 of the core element 21f. At the same time, the absorbing component 3 is folded along the grooves 20s, 20t so that the inner surface 23 of the core element 21a is opposed to the inner surfaces 23 of the respective core elements 21c, 21d while the inner surface 23 of the core element 21b is opposed to the inner surfaces 23 of the respective core elements 21e, 21f (See Fig. 4). The grooves 20r, 20s, 20t serve an folding guides each extending from the transversely middle zone to each of the lateral edges 26 of the absorbing component 3 so as to describe substantially V-shape in the portions of placed aside toward its front and rear ends, respectively. In the grooves 20r, 20s, 20t, there is little or no core 21. So the absorbing component 3 has its stiffness lower in these grooves 20r, 20s, 20t than in the other region. This unique arrangement facilitates the absorbing component 3 to be folded along the grooves 20r, 20s, 20t as shown in Fig. 2.

In the pull-on disposable diaper 1 of Fig. 1 arranged as has been described, the absorbing component 3 is folded at the opposite lateral edges 26, 26 so that the width of the absorbing component 3 may be reduced in the crotch covering region 8 and those opposite lateral edges 26, 26 extend upward. Accordingly, the diaper 1 comes in close contact with the wearer's crotch in the vicinity of the wearer's genital organs and an amount of urine is absorbent rapidly into the diaper 1 without spreading sideways. In addition, a space defined between these opposite lateral edges 26, 26 forms a pocket 30a depressed downward (See Fig. 3) serving to prevent a possibility that the inner surface 3a (See Fig. 5) of the absorbing component 3 wetted with absorbed urine might come in contact with the wearer's body over a large area and create an uncomfortable damp feeling against the wearer. Even if the body fluid flows beyond the lateral edges 26, the lateral marginal edges 8a of the crotch covering region 8 extending outward beyond the lateral edges 26 and defining the flaps 11a adapted to be tightened around the wearer's thighs function as barriers against leakage of the body fluids from the diaper 1. Furthermore, the outer surface 24 of the core 21 is covered with the liquid-pervious cover sheet 22 at least in the vicinity of the side surfaces 40a, so if any amount of body fluids flow beyond the peripheral edge of the absorbing component 3, such amount of body fluids will be introduced into outer pockets 30b (See Fig. 3) formed between the absorbing component 3 and the respective lateral marginal edges 8a of the crotch covering region 8 and absorbed by the core 21 through its outer surface 24. It should be noted here that the outer surface 24 of the core 21 may be at least partially, for example, in its transversely middle zone, covered with the liquid-impervious intermediate sheet 17 as shown in Figs.

In this diaper 1, as will be apparent from Figs. 3, 4, 5, the lateral edge zones 22a of the cover sheet 22 are folded outward in the transverse direction of the absorbing component 3 and joined to the inner sheet 16 by means of the adhesive agent 29 and thereby movement of the core's region having been folded along the groove 20r to restore the state before folded, in other words, movement outward with respect to the diaper 1 is restrained. The movement outward with respect to the diaper is the movement in the direction indicated by the arrow Y in Fig. 3. It is believed that the movement of the region extending along the groove 20r in the direction of the arrow Y is restrained because the core elements 21c, 21e get nearer to each other and the core elements 21d, 21f get nearer to each other as the lateral edge zones 22a is deformed from its flat state as seen in Fig. 4 to its curved state as seen in Fig. 3. So far as such movement is restrained, the absorbing component 3 folded so as to become narrower in the crotch covering region 8 can be reliably kept in this folded configuration even after the diaper has been put on the wearer' s body.

To put the diaper 1 on infant's body, his or her mother may guide the infant's legs through the waist-hole 9 opened as widely as possible with the mother's hands put against the inner side of the waist-hole 9 into the respective leg-holes 11. With this diaper 1, the absorbing component 3 provided separately of the covering component 2 in the crotch covering region 8 far from the waist-hole 9 is not affected by deformation of the diaper 1 occurring as the waist-hole 9 is opened in this manner. Furthermore, even if the lateral marginal edges 8a of the crotch covering region 8 moves due to broadening of the waist-hole 9, there is substantially no possibility that the absorbing component 3 gets out of its initial position since the lateral edges 26 of the absorbing component 3 are not joined to the lateral marginal edges 8a. In this way, there is not likely that the absorbing component 3 folded so as to be narrower in the crotch covering region 8 might get out of its desired shape and the lateral edges 26 of the absorbing component 3 extending upward are reliably brought in close contact with the infant's crotch. Therefore, the absorbing component 3 folded so as to reduce its width reliably provides its expected effect when the diaper 1 is put on the infant' s body. Without departing from the scope of the invention, it is possible to eliminate provision of the groove 20r depending on a thickness of the core 21 and to fold the absorbing component 3 using the grooves 20s, 20t. It is also possible to locally press with or without heating the zones of the absorbing component 3 extending along the chain lines R, S, T or the chain lines S, T using embossing rolls or the like so that these zones may have stiffness higher than that in the other zone and the absorbing component 3 may be folded in parallel to these zones of relatively high stiffness.

The pull-on disposable diaper according to this invention has advantageous effects that the body fluid absorbing component folded inward in the transverse direction of the core in the crotch covering region to reduce the width of the core can be reliably maintained in such a folded state because the body fluid absorbing component comprising the body fluid absorbing core and the liquid-pervious cover sheet have the transversely opposite lateral marginal edges folded back on the outer surface of the core outward in the transverse direction of the core.

## Claims

1. A pull-on disposable diaper having a height direction, a width direction being orthogonal to said height direction and a back-and-forth direction, said diaper being substantially symmetric about a center line bisecting a dimension of said diaper in said width direction, said diaper comprising a covering component made of sheet material having an inner surface facing a wearer' s body and outer surface facing a wearer's clothes and composed of front waist covering region destined to cover a wearer' s front waist, a rear waist covering region destined to cover a wearer's rear waist and a crotch covering region destined to cover a wearer's crotch so as to define a pants-like configuration having a waist-hole and a pair of leg-holes, and a body fluid absorbing component extending over said crotch covering region and further extending into said front and rear waist covering regions, said pull-on disposable diaper further comprising:
said body fluid absorbing component comprising a liquid-absorbent core and a liquid-pervious cover sheet, said core having an inner surface facing said wearer' s body, an outer surface facing away from said wearer' s body and side surfaces extending between said inner and outer surfaces in said height direction and extending beyond said crotch covering region into said front and rear waist covering regions, said cover sheet covering said inner surface, said side surfaces and at least a part of said outer surface of said core, said body fluid absorbing component being formed in said crotch covering region with a first folding guide extending from a middle zone between transversely opposite lateral edges extending parallel to each other in said height direction to respective said lateral edges in said front waist covering region so as to describe a substantially V-shape, a second folding guide extending to respective said lateral edges in said rear waist covering region so as to describe a substantially V-shape and a third folding guide extending in said width direction between said first and second folding guides, and said absorbing component being folded on both sides of said center line along said third folding guide so that said core has its outer surface sections facing to each other and along said first and second folding guides so that said core has its inner surface sections facing to each other, transversely opposite lateral edges of said cover sheet being folded back outward in said width direction on said outer surface of said core and being joined to the inner surface of said sheet material defining said crotch covering region.

2. The disposable diaper according to Claim 1, wherein said outer surface of said core is covered at least with said cover sheet and a liquid-impervious sheet lying outside said cover sheet and defining said crotch covering region.

3. The disposable diaper according to Claim 2, wherein said outer surface of said core is covered with said cover sheet, a nonwoven fabric lying outside said cover sheet, a plastic film lying outside said nonwoven fabric and defining said liquid-impervious sheet and a nonwoven fabric lying outside said liquid-impervious sheet.

## Patentansprüche

1. Wegwerf-Höschenwindel mit einer Höhenrichtung, einer zu der Höhenrichtung senkrechten Breitenrichtung und einer Vorwärts-Rückwärtsrichtung, welche Windel im wesentlichen um eine eine Abmessung der Windel in der Breitenrichtung halbierende Mittellinie symmetrisch ist, wobei die Windel eine Bedeckungskomponente aufweist, die aus Lagenmaterial hergestellt ist, welches eine zum Körper des Trägers weisende innere Oberfläche und eine zu der Bekleidung des Trägers weisende äußere Oberfläche hat und
aus einem vorderen Hüftbedeckungsbereich, der zum Bedecken des vorderen Hüftbereichs eines Trägers bestimmt ist, einem hinteren Hüftbedeckungsbereich, der zum Bedecken eines hinteren Hüftbereichs eines Trägers bestimmt ist, und einem Schrittbedeckungsbereich zusammengesetzt ist, der zum Bedecken eines Schrittbereichs eines Trägers bestimmt ist, um so eine höschenartige Konfiguration zu bilden, die eine Hüftöffnung und einen Paar Beinöffnungen hat, sowie eine Körperflüssigkeit absorbierende Komponente, die sich über den Schrittbedeckungsbereich erstreckt und welter in den vorderen und
den hinteren Hüftbedeckungsbereich verläuft, welche Wegwerf-Höschenwindel ferner enthält:
die Körperflüssigkeit absorbierende Komponente, welche einen flüssigkeitsabsorbierenden Kern und eine flüssigkeitsdurchlässige Decklage aufweist, welcher Kern eine zum Körper des Trägers weisende innere Oberfläche hat, eine vom Körper des Trägers abgewandte äußere Oberfläche sowie Seitenflächen, die zwischen der inneren und der äußeren Oberfläche In der Höhenrichtung verlaufen und über den Schrittbedeckungsbereich in den vorderen und den hinteren Hüftbedeckungsbereich verlaufen, welche Decklage die innere Oberfläche, die Seitenflächen und mindestens einen Tell der äußeren Oberfläche des Kerns bedeckt, welche Körperflüssigkeit absorbierende Komponente In dem Schrittbedeckungsbereich mit einer ersten Faltführung, die von einer mittleren Zone zwischen in Querrichtung einander gegenüberliegenden Seitenrändern, die In der Höhenrichtung parallel zueinander verlaufen, zu den jeweiligen Seitenrändern in dem vorderen Hüftbedeckungsbereich verläuft, so dass sie im wesentlichen eine V-Form beschreibt, einer zweiten Faltführung, die zu den jeweiligen Seltenrändern In dem hinteren Hüftbedeckungsbereich verläuft, so dass sie Im wesentlichen eine V-Form beschreibt, und einer dritten Faltführung gebildet Ist, die in der Seitenrichtung zwischen der ersten und der zweiten Faltführung verläuft, und wobei die absorbierende Komponente auf beiden Seiten der Mittellinie entlang der dritten Faltführung gefaltet Ist, so dass die äußeren Oberflächenabschnitte des Kerns aufeinander zu weisen, und entlang der ersten und der zweiten Faltführung, so dass die Inneren Oberflächenabschnitte des Kerns aufeinander zu weisen, wobei in Querrichtung einander gegenüberliegende Seltenränder der Decklage in der Breitenrichtung auf die äußere Oberfläche des Kerns nach außen zurück gefaltet sind und mit der Inneren Oberfläche des Lagenmaterials verbunden sind, welches den Schrittbedeckungsbereich bildet.

2. Wegwerfwindel nach Anspruch 1, bei welcher die äußere Oberfläche des Kerns mindestens mit der Decklage und einer flüssigkeitsundurchlässigen Lage bedeckt ist, die außerhalb der Decklage liegt und den Schrittbedeckungsbereich bildet.

3. Wegwerfwindel nach Anspruch 2, bei welcher die äußere Oberfläche des Kerns mit der Decklage, einem außerhalb der Decklage liegenden Vliesstoff, einer außerhalb des Vliesstoffes liegenden Kunststofffolie, welche die flüssigkeitsundurchlässige Lage bildet, und einem außerhalb der flüssigkeitsundurchlässigen Lage liegenden Vliesstoff bedeckt ist.

## Revendications

1. Couche jetable à taille élastique ayant une direction en hauteur, une direction en largeur orthogonale à ladite direction en hauteur et une direction avant et arrière, ladite couche étant essentiellement symétrique autour d'une ligne centrale coupant une dimension de ladite couche dans ladite direction de largeur, ladite couche comprenant un composant couvrant fait d'un matériau de feuille ayant une surface interne faisant face au corps du porteur et une surface externe faisant face au vêtement du porteur et composée d'une région couvrante avant de taille destinée à couvrir une taille avant de porteur, une région couvrante arrière de taille destinée à couvrir une taille arrière de porteur et une région couvrante d'entrejambe destinée à couvrir un entrejambe de porteur de manière à définir une forme de culotte ayant un trou de taille et une paire de trous de jambes, et un composant absorbant le liquide corporel s'étendant sur ladite région couvrante d'entrejambe et s'étendant plus loin dans lesdites régions avant et arrière de taille, ladite couche jetable à taille élastique comprenant en outre:
ledit composant absorbant le liquide corporel comprenant un coeur absorbant le liquide et une feuille couvrante perméable au liquide, ledit coeur ayant une surface interne faisant face audit corps de porteur, une surface externe opposée audit corps de porteur et des surfaces latérales s'étendant entre lesdites surfaces interne et externe dans ladite direction en hauteur et s'étendant au-delà de ladite région couvrante d'entrejambe dans les dites régions arrière et avant couvrantes de taille, ladite feuille couvrante recouvrant ladite surface interne, lesdites surfaces latérales et au moins une partie de ladite surface externe dudit coeur, ledit composant absorbant le guide corporel étant formé dans ladite région couvrante d'entrejambe avec un premier guide de pliage s'étendant depuis une zone médiane entre des bords latéraux opposés transversalement s'étendant parallèlement entre eux dans ladite direction en hauteur vers lesdits bords latéraux respectifs dans ladite région couvrante de taille de manière à décrire une forme essentiellement en V, un second guide de pliage s'étendant en direction desdits bords latéraux respectifs dans ladite région avant de taille couvrante de manière à décrire une forme essentiellement en V et un troisième guide de pliage s'étendant dans ladite direction de largeur entre lesdits premier et second guides de pliage, et ledit composant absorbant étant plié des deux côtés de ladite ligne centrale le long dudit troisième guide de pliage de sorte que ledit coeur a ses sections de surface externes qui se font face mutuellement et situées le long desdits premier et second guides de pliage de sorte que ledit coeur a ses sections de surface internes se faisant face mutuellement, des bords latéraux transversalement opposés de ladite feuille couvrante étant repliés vers l'extérieur dans ladite direction en largeur sur ladite surface externe dudit coeur et étant reliés à la surface Interne dudit matériau de feuille définissant ladite région couvrante d'entrejambe.

2. Couche-culotte jetable selon la revendication 1, dans laquelle ladite surface externe dudit coeur est couverte au moins par ladite feuille couvrante et une feuille imperméable au liquide reposant à l'extérieur de ladite feuille couvrante et définissant ladite région couvrante d'entrejambe.

3. Couche-culotte jetable selon la revendication 1, dans laquelle ladite surface externe dudit coeur est recouverte par ladite feuille couvrante, un tissu non-tissé se trouvant à l'extérieur de ladite feuille couvrante, un film plastique se trouvant à l'extérieur dudit tissu non tissé et définissant ladite feuille imperméable au liquide, et un tissu non tissé se trouvant à l'extérieur de ladite feuille imperméable au liquide.
